(19) Europäisches Patentamt / European Patent Office / Office européen des brevets

(11) **EP 3 543 726 A1**

(12) **EUROPEAN PATENT APPLICATION**

(43) Date of publication:
25.09.2019 Bulletin 2019/39

(51) Int Cl.:
*G01R 33/58* (2006.01)    *G01R 33/48* (2006.01)

(21) Application number: 19164169.5

(22) Date of filing: 20.03.2019

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 23.03.2018 GB 201804702

(71) Applicant: **Elekta Ltd.**
**Crawley RH10 9RR (GB)**

(72) Inventors:
• **BOURNE, Duncan Neil**
**Redhill, Surrey RH1 3BA (GB)**
• **SHAIKH, Mohammad**
**Crawley, Sussex RH10 2RR (GB)**

(74) Representative: **Downing, Michael Philip**
**Downing IP Limited**
**Grosvenor House**
**7 Horseshoe Crescent**
**Beaconsfield, Bucks. HP9 1LJ (GB)**

(54) **MARKER FOR RADIOTHERAPY APPARATUS COMPRISING BUBBLE CHAMBER**

(57)    We have noticed that during assembly of a marker according to our previous application GB2512416, i.e. one having a solid first component and a liquid second component, a small amount of air is sometimes trapped within a housing of the marker. Precautions can obviously be taken to limit this, but it would be preferable if the structure was resilient to this. Accordingly, the present invention is directed to a marker for use in calibration of a radiotherapeutic apparatus, comprising a first component, preferably spherical, and having a first hydrogen proton density and a first mass density, and being solid, a second component having a second hydrogen proton density and a second mass density, and being liquid, wherein the first component is substantially completely surrounded by the second component within a void of the second component to form an interface between the first and second components, the marker further comprising a housing enclosing the first and second components, and defining a region into which the second component can extend at a location which is displaced vertically from the interface between the first and second components, thereby to allow trapped air to be collected. Thus in use any trapped air is encouraged to migrate to a position away from the first component, allowing the interface between the first and second components to be free of contamination from such trapped air.

Fig 3

**Description**

FIELD OF THE INVENTION

The present invention relates to medical imaging, and particularly to markers, phantoms, and associated methods of calibrating an imaging system which may be integrated with a radiotherapy system.

BACKGROUND ART

Our earlier patent application GB2512416 discloses a marker for use in calibration of a medical imaging system comprising a first component having a first hydrogen proton density and a first mass density and a second component having a second hydrogen proton density different than the first hydrogen proton density and a second mass density different than the first mass density, wherein the first and second components are non-magnetic. The first component is generally located within a void of the second component so that the first component is substantially entirely surrounded by the second component. Such a marker can provide a well-defined interface between the first and second components when imaged by both magnetic resonance imaging (MRI) apparatus and linear-accelerator-based imaging or treatment apparatus such as X-ray apparatus and the like.

The provision of a well-defined interface which responds to both types of imaging system in a marker enables calibration and co-ordinate co-registration of imaging radiation and treatment radiation in radiotherapy systems which include both MRI and linac systems.

Our earlier patent application disclosed possible materials for the first component being ceramics such as zirconium oxide (zirconia), and possible materials for the second component being oils such as cod-liver oil or oil-based solids, or water-based solutions.

One advantage of an arrangement as disclosed by our earlier application is that by surrounding a solid first component with a liquid second component one can reduce the risk of the presence of any other material between the outer surface of the first component and the liquid of the second component to substantially zero. Thus, for example, such an arrangement may provide a more precise relationship between the first and second components than if both were selected from solid materials and moulded or machined before being assembled into a marker.

SUMMARY OF THE INVENTION

We have noticed that during assembly of a marker having a solid first component and a liquid second component a small amount of air is sometimes trapped within a housing of the marker. Image analysis software will be looking for the interface between the components, defined by the shape of the first component, and irregularities in that interface will be undesirable. Precautions can obviously be taken to limit inclusion of air, but it would be preferable if the structure was resilient to this.

Accordingly, the present invention is directed to a marker for use in calibration of a radiotherapeutic apparatus, comprising a first component, preferably spherical, and having a first hydrogen proton density and a first mass density, and being solid, a second component having a second hydrogen proton density and a second mass density, and being liquid, wherein the first component is substantially completely surrounded by the second component within a void of the second component to form an interface between the first and second components, the marker further comprising a housing enclosing the first and second components, and defining a region into which the second component can extend at a location which is displaced vertically from the interface between the first and second components, thereby to allow trapped air to be collected - ideally away from the first component.

Thus in use any trapped air is encouraged to migrate to a position away from the first component, allowing the interface between the first and second components to be free of contamination from such trapped air. This provides an improved and more predictable contrast between the first and second components under both radiation based imaging modalities and magnetic resonance based imaging modalities.

The region is provided in a location above the first component when the marker is in use. Thus, when the second component is more dense than ambient air, any trapped air will tend to rise towards that region under gravity.

The housing may comprise a tapered portion, which allows for a region that is also laterally displaced from the interface between the first and second components thus ensuring a greater distance between the interface and any air bubbles.

The housing may comprise more than one such region. The housing can further comprise a restraint for restraining the first component in a pre-determined position within the housing. The restraint may be formed by a shape of the housing, such as a taper into which the first component fits. Alternatively or in addition, the restraint may comprise a portion which extends into the marker. For example, the restraint may provide a region of the marker which is narrower than the housing. Such an arrangement could prevent movement of the first component in a particular direction within the marker. One example of this arrangement may be one or more fingers or tongues extending from the housing into the marker, to allow flow of the second component or indeed trapped air around it or them while inhibiting movement of the first component.

The restraint may form a cage substantially around the first component, in use.

A marker may comprise a housing having two or more traps arranged on either side of the first component in use. A trap on each side of the first component may provide for the collection of air trapped on either side of the first component.

A marker may have more than one first components positioned within the marker. The first components may be positioned in pre-determined locations within the marker. Thus, the relative locations of the plurality of first components may provide further information to a suitable calibration process. A marker having more than one first component may further have more than one trap. A marker having more than one first component may further have more than one restraint.

BRIEF DESCRIPTION OF THE DRAWINGS

An embodiment of the present invention will now be described by way of example, with reference to the accompanying figures in which;

Figure 1 shows a marker according to our earlier patent application GB2512416;

Figure 2a is a schematic illustration of an MR image of the marker of Figure 1;

Figure 2b is a schematic illustration of a radiographic image of the same marker;

Figure 3 shows a marker according to a first embodiment of the present invention;

Figure 4 shows a marker according to a second embodiment of the present invention;

Figure 5 shows a marker according to a third embodiment of the present invention;

Figure 6a shows a phantom according to embodiments of the present invention;

Figure 6b shows a phantom according to further embodiments of the present invention;

Figure 7 shows a combined MRI radiotherapy system; and

Figure 8 is a flowchart of a method according to embodiments of the present invention.

DETAILED DESCRIPTION OF THE EMBODIMENTS

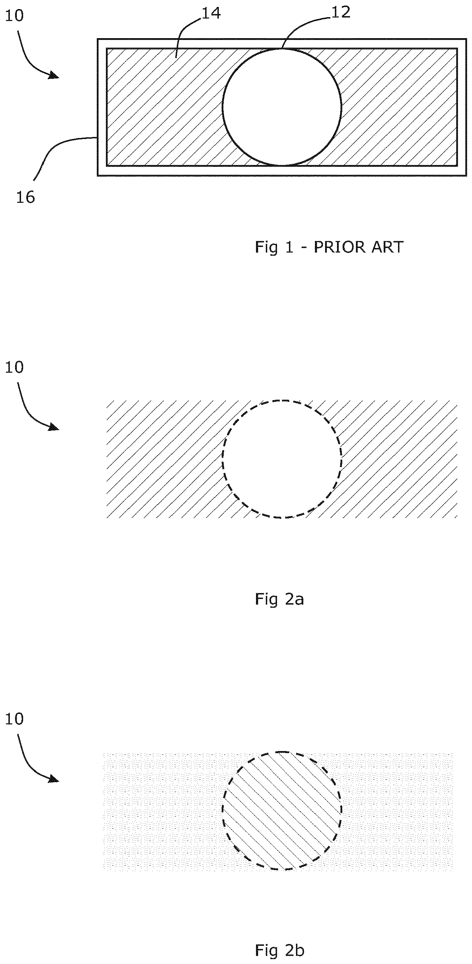

Figure 1 shows a cross section of a marker 10 according to our earlier patent application GB2512416. As will be explained below, the illustrated marker is suitable for use in a variety of medical imaging systems using different imaging modalities.

In order to understand how the marker works, it is instructive first to consider the different imaging mechanisms which may be employed in medical imaging.

Magnetic resonance (MR) imaging operates by placing the imaging object in a high strength magnetic field. Currently, the field strength density typically varies from system to system between 0.2 and 3 T. In this strong magnetic field, the magnetic moments of hydrogen protons in the object become aligned with the magnetic field.

By applying an electromagnetic signal having a resonant frequency to the object, the spins of those protons can be made to flip. When the electromagnetic signal is switched off, the protons flip back and emit an electromagnetic signal which can be received in receiver coils. Gradient magnets are employed to vary the magnetic field spatially, so as to generate detectable signals only from certain locations within the object and/or to make the resonant frequency differ at different locations to enable spatial encoding of received electromagnetic signals. Hydrogen protons in different materials return to their equilibrium state at different relaxation rates, and this can also be used to differentiate between materials and construct images.

In this way, materials with high hydrogen proton densities, i.e. materials with high numbers of hydrogen protons which are free to flip their magnetic moment, are clearly and strongly visible in MR images.

Another imaging modality employs radiation such as x-rays. An object to be imaged is targeted with a collimated x-ray beam; typically the beam is cone-shaped, but other shapes could be employed. A detector positioned on the opposite side of the body detects the radiation after it has passed through the object. Some of the radiation will have been absorbed by the object, such that the data collected by the detector provides information on the location of the object in the form of a projection image. Multiple projection images can be combined to reconstruct a volume image of the object using computed tomography (CT) techniques.

The likelihood of an object absorbing x-rays of a given energy increases with increasing material density of the object, although the increase is not linear. High-density materials such as lead or tungsten absorb x-rays very readily, which leads to them being employed in collimators, radiation shields and the like. Low-density objects may not be visible in radiation-based images. The likelihood of absorption also depends on the energy of the radiation, with different mechanisms of interaction dominating at different energies. For example, in the case of lower energy x-rays (i.e. kV range), in addition to the effects of material density, x-ray absorption can be quite material sensitive due to the photoelectric effect. Different materials absorb kV x-rays differently (e.g. as seen in the clear imaging of bone in kV x-rays). However, at higher energy levels (i.e. MV range) the relative absorption depends mainly on the relative material density of the materials in the object. At MV energy levels, therefore, a high contrast image can be obtained by imaging materials with different material densities. The greater the difference in material density, the greater the contrast in the image.

The marker 10 according to embodiments of the present invention can be employed in imaging systems employing these and other techniques.

The marker 10 comprises a first marker component 12 which is solid and, in the illustrated embodiment, has a spherical shape. The first marker component 12 is non-magnetic, in that it has no significant effect on an external magnetic field (i.e. it is non-ferromagnetic). This allows the marker 10 to be employed in magnetic resonance (MR) imaging systems. Further, the first marker component 12 is ideally non-conductive, as conductive materials can cause distortion in MR images. In the context of the present invention, a component can be deemed non-conductive if the radio frequency field generated by an MRI system can penetrate (i.e. pass through) the component. The skin depth of the component material at such frequencies must therefore be substantially equal to or greater than the size of the component itself (the skin depth $\delta$ is given by $\delta = \sqrt{\dfrac{2\rho}{\omega\mu}}$ where $\rho$ is the resistivity, $\omega$ is the angular frequency and $\mu$ is the absolute magnetic permeability). For example, in a 1.5 T MRI the rf field frequency (the resonant frequency of hydrogen protons) will be at or around 64 Mhz. Given this information, it is a simple exercise for the skilled person to select a suitable material to ensure that the skin depth is equal to or greater than the size of the component.

The first marker component 12 has a relatively high material density, so that it preferentially absorbs x-rays and appears in radiation-based images, i.e. radiographic images using x-rays. However, the first marker component 12 also has a relatively low hydrogen proton density, so that it appears only weakly in MR images. In some embodiments, the first marker component 12 has substantially zero hydrogen proton density so that it is not imaged in MR imaging systems.

One class of material which may be suitable for use in the first marker component is ceramic materials, as they are non-magnetic and non-conductive. Certain ceramic materials also have a high material density, such as zirconium oxide (zirconia), which has a material density of 5.66 kgm$^{-3}$.

The marker 10 further comprises a second marker component 14. The second marker component 14 is also non-magnetic (i.e. non-ferromagnetic), but has a relatively low material density (i.e. relative to that of the first marker component) and a relatively high hydrogen proton density (again, relative to that of the first marker component).

In this way, the second marker component 14 is imaged more strongly than the first marker component 12 in MR imaging techniques, while the first marker component 12 is imaged more strongly than the second marker component 14 in radiographic techniques (i.e. in both MRI and radiographic images, a contrast is achieved between the two components).

In the illustrated arrangement, the second marker component 14 surrounds the first marker component 12, but in other arrangements the second marker component 14 may only be in contact with the first marker component 12 on part of its surface such that, effectively, the two components have at least one common surface. In order that the second marker component 14 can easily conform to the surface of the first marker component 12, the second marker component 14 is liquid.

The second marker component may also be non-conductive, in that an rf field will penetrate the component as described above. For example, the second marker component 14 may comprise an oil, such as cod liver oil, or an oil-based solid. In alternative arrangements the second marker component 14 may comprise water or a water-based solution.

The marker 10 further comprises a housing 16 which substantially surrounds and supports the other components of the marker. In one embodiment the housing 16 supports the first marker component 12 at a fixed position within the housing. The second marker component 14 substantially fills any voids in the housing 16 not filled by the first marker component 12. The housing 16 may be manufactured from a plastic, such as poly(methyl methacrylate) - otherwise known as Perspex (RTM) - and may be transparent or opaque to visible optical light. The housing 16 is non-magnetic. It may also have a substantially zero hydrogen proton density and therefore be substantially invisible to MR imaging modalities.

The first marker component 12 and/or the housing 16 may take a non rotationally symmetric shape such that their orientation can be determined more accurately when imaged. A spherical object can be easily imaged and measured, but cannot provide information on the orientation of the marker 10. In certain situations that may not matter, and the ease of detection of a spherical marker may be more useful. However, a non-rotationally symmetric first marker component 12 and/or housing 16 can provide that information.

In further embodiments, the marker 10 may comprise multiple first marker components 12 within the housing 16, each of which can he imaged positively using radiation-based techniques and negatively using MR-based techniques.

Figures 2a and 2b are schematic illustrations of MR-based and radiation-based images respectively of the marker 10 shown in Figure 1.

In Figure 2a (MR imaging), the second marker component 14 is visible due to its high hydrogen proton density while the first marker component 12 is less visible (or even invisible) due to the relatively low hydrogen proton density. However, due to the common surface between the two components - shown in Figure 2a as a dashed line - and the contrast between the two materials in the image, the first marker component 12 can nonetheless be visualized. That is, the spherical space within the second marker component 14 corresponds to the first marker component 12, and therefore the first marker component can be effectively imaged using MR techniques.

In Figure 2b (radiography), the first marker component 12 is directly visible as it has a higher material density that the second marker component 14. The second marker component 14 also has a finite material density and therefore also appears in the image. However, due to the difference in material densities, the common surface of the two components (again shown by a dashed line) can be imaged. The centre of the first marker component 12 can therefore act as a common reference point in radiographic and MR images.

Note that the appearance of the first and second marker components in the radiographic image depends on a number of factors, including the energy of the radiation and the quantity of the radiation (i.e. as determined by the intensity of the beam and the exposure time). A small amount of radiation may result in the second marker component 14 effectively being invisible in the radiographic image. However, it is sufficient that only the first marker component 12 is visible, and that a boundary between the two components can be discerned.

In some embodiments it may desirable to decouple the size of the effective marker as imaged using radiographic techniques from the effective size of the marker as imaged using MRI techniques. For example, a smaller first marker component 12 (i.e. the radiation absorbing component) increases the accuracy of calibration of a radiation based device, whilst use of a relatively large non-magnetic, low density material preserves the accuracy of calibration of an MRI device.

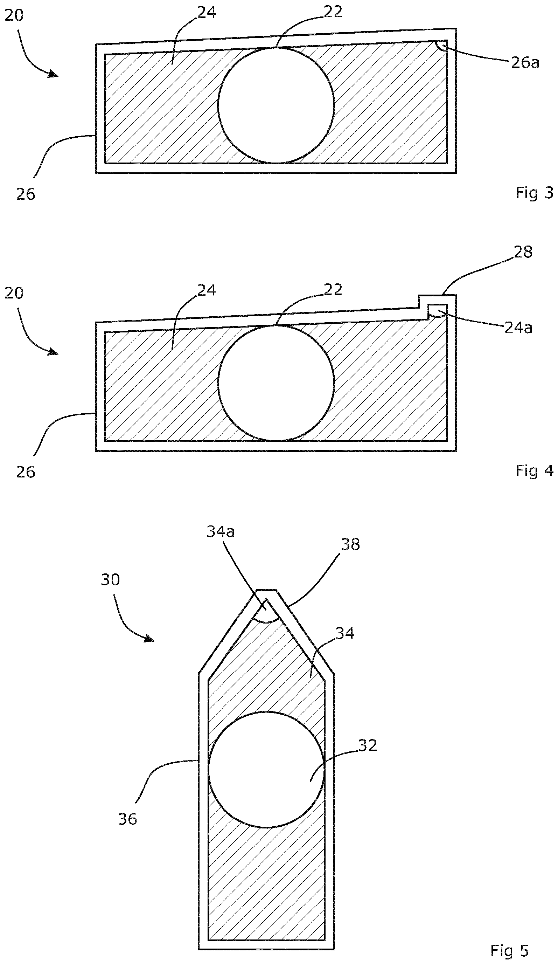

Figure 3 shows a cross section of a marker 20 according to embodiments of the present invention. The marker 20 may be employed in an equivalent manner to that of the marker 10 shown in Figure 1. The marker 20 comprises a first marker component 22 having equivalent material properties to the first marker component 12 described with reference to Figure 1, i.e. relatively high material density such that it appears in radiation-based images and relatively low hydrogen proton density so that it appears only weakly in MR images.

The marker 20 further comprises a second marker component 24. The second marker component 24 is equivalent to the second marker component 14 of the marker 10 described with reference to Figure 1. In other words, the second marker component 24 is non-magnetic (i.e. non-ferromagnetic), of relatively low material density and has a relatively high hydrogen proton density. Accordingly, the second marker component 24 is imaged more strongly than the first marker component 22 using MR imaging techniques, while the first marker component 22 is imaged more strongly than the second marker component 24 using radiographic techniques.

In common with the marker described with respect to Figure 1, the coincident centre point of the first marker component 22 and the negative space delineated by the second marker component 24 may be used as a common geometric reference point.

The marker 20 further comprises a housing 26 equivalent to the housing 16 shown in Figure 1 which substantially surrounds and supports the other components of the marker 20. In one embodiment the housing 26 supports the first marker component 22 at a fixed position within the housing. The second marker component 24 substantially fills the void in the housing 28 around and otherwise unfilled by the first marker component 22. The housing 28 may be manufactured from a plastic, such as Perspex (RTM) and may be transparent or opaque to visible optical light. It may also have a substantially zero hydrogen proton density and therefore he substantially invisible to MR imaging modalities.

In further embodiments, the marker 20 may comprise multiple first marker components 22 within the housing 26.

Figure 1 showed a housing 16 that was symmetric around its long axis. In contrast, the housing 26 of figure 3 shows a slight taper along its length, with an internal dimension that matches the first marker component 22 approximately halfway along its length. This will result in there being an upper corner 26a of the housing which, when the marker is in position and level, will be vertically higher than the first marker component 22. Assuming that the second component 24 is more dense than air, any air or other gases that are trapped within the housing 26 will migrate under gravity into that upper corner 26a, as shown. This therefore defines a collection region for trapped air. As the housing is tapered, this region is also spaced laterally from the interface between the first and second components, thereby moving trapped air (etc) away from the interface and assisting in defining a clear interface.

Figure 4 illustrates a second embodiment with a distinct trap 24a. This embodiment is similar to that of figure 3 but at the widest end of the housing 26 there is a lateral protrusion 28 which defines a recess 24a in the space otherwise occupied by the second marker component 24. In use, the protrusion 28 will be oriented upwardly so that any bubbles in the second marker component 24 will be guided by the taper of the housing 26 and rise into the recess 24a, which therefore acts as a trap for the bubbles.

As a result the design of both embodiments, any bubbles remaining after the housing 26 is filled or which form from gases dissolved in the second marker component 24 are safely confined away from the first marker component 22. Imaging of the device will then reveal a sharp delineation between the marker components without such types of interference.

Figure 5 shows a cross section of a marker 30 according to a further embodiment of the present invention. The marker 30 may be employed in an equivalent manner to that of the marker 10 shown in Figure 1 or the marker 20 shown in Figure 3. The marker 30 comprises a first marker component 32 having equivalent material properties to the first marker component 12 described with reference to Figure 1, i.e. relatively high material density such that it appears in radiation-based images and relatively low hydrogen proton density so that it appears only weakly in MR images.

The marker 30 further comprises a second marker component 34. The second marker component 34 is equivalent to the second marker component 14 of the marker 10 described with reference to Figure 1. In other words, the second marker component 34 is non-magnetic (i.e. non-ferromagnetic), of relatively low material density and has a relatively high hydrogen proton density. Accordingly, the second marker component 34 is imaged more strongly than the first marker component 32 using MR imaging techniques, while the first marker component 32 is imaged more strongly than the second marker component 34 using radiographic techniques.

In common with the examples described with respect to Figures 1 and 3, the coincident centre point of the first marker component 32 and the negative space delineated by the second marker component 34 may be used as a common geometric reference point.

The marker 30 further comprises a housing 36 equivalent to the housing 16 shown in Figure 1 which substantially surrounds and supports the other components of the marker 20. The second marker component 34 substantially fills the void in the housing 36 around and otherwise unfilled by the first marker component 32. The housing 36 may be manufactured from a plastic, such as Perspex (RTM) and may be transparent or opaque to visible optical light. It may also have a substantially zero hydrogen proton density and therefore be substantially invisible to MR imaging modalities.

As illustrated in figure 5, the marker 30 is oriented upright, so that the second marker component 34 occupies a space above and below the first marker component 32. The upper extremity of the housing 36 has a conical shape 38 so as to define a recess 34a into which bubbles in the second marker component 34 can rise, although it need not and may be flat. Thus, as with the marker 20, the marker 30 is resilient to the formation of bubbles in the second marker component 34 as they are guided away from the interface with the first marker component 32 and hence do not affect imaging of that interface.

In both the marker 20 described with reference to figures 3 and 4, and the marker 30 described with reference to figure 5, grooves or passageways may be formed in the internal faces of the housings 26, 36 around at least the area occupied by the first marker component 22, 32 in order to allow movement of bubbles past the first marker component 22, 32, towards the recess 24a, 34a. Alternatively, or in addition, multiple regions 24a, 26a may be provided at locations on either side of the first marker component.

In further embodiments, the first marker component may have a lower material density than the second marker component. As long as there is a difference between the two marker components in hydrogen proton density and material density, a contrast is achieved that allows the marker components to be imaged. It will however be appreciated that for radiation imaging, a relatively small marker component is preferable and for MR imaging, a relatively large marker component is desirable.

As described above, phantoms are known devices which are scanned or imaged to evaluate and tune the performance of various medical imaging devices. In one embodiment, either of the markers 20, 30 themselves can be employed as a phantom.

That is, the dimensions of the marker 20 and of the first marker component 22 maybe made sufficiently large that an image of the marker on its own provides sufficient accuracy to enable the imaging system to be calibrated. For example, if the first marker component 22 has a largest dimension in the order of 100 mm, the marker 20 may be placed in the imaging system on its own as part of the calibration process.

Equally, if the first marker component 32 of the marker 30 has a largest dimension in the order of 100 mm, for example, the marker 30 maybe placed in the imaging system on its own as part of the calibration process. Alternatively, a marker 20, 30 as described above and comprising multiple first marker components may he used on its own as a phantom.

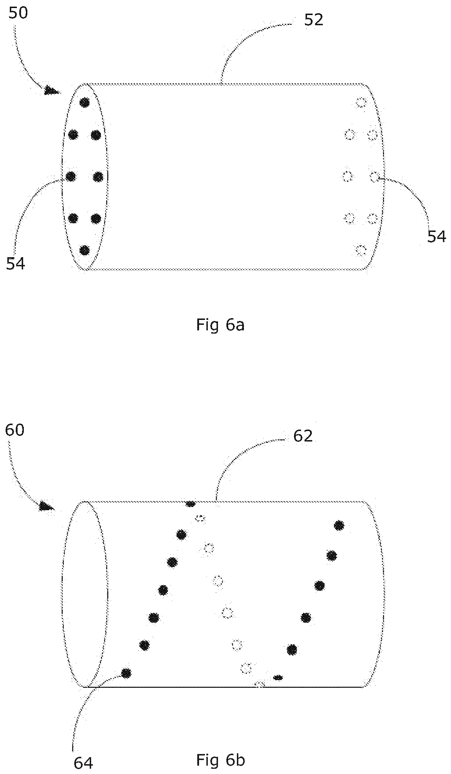

However, sourcing and producing such a large device may be impractical due to issues of cost and efficiency. In other embodiments of the invention, either of the markers 20, 30 may be produced on a smaller scale such that the first marker components 22, 32 have a largest dimension in the order of 10 mm. In this way, conventional phantoms can be adapted to include a plurality of such markers arranged in one or more patterns. Figures 6a and 6b show examples of such phantoms.

Figure 6a shows a phantom 50. The phantom has a housing 52 which may be manufactured from a non-magnetic, non-conductive material such as plastic. In the illustrated embodiment the housing is cylindrical but in practice any shape may be employed. The housing has a number of attaching points (not illustrated), to which the markers 54 corresponding to either the markers 20 shown in Figure 3 or 4 and/or the markers 30 shown in Figure 4 are attached, or any other marker being an embodiment of the invention. Any means of attaching the markers 54 to the housing 52 can be used. For example, slots or openings may be provided in the housing, into which the markers 54 can be inserted. In this example, the markers 54 are arranged in two circles, one at each end of the cylindrical housing.

Figure 6b shows a further example of a phantom 60 according to embodiments of the invention. In this example, the markers 64 are arranged in a helical pattern around the housing 62.

In either case, the pattern of markers 54, 64 allows the phantom's location and orientation to be precisely measured.

It will be apparent from the description above that markers and phantoms according to embodiments of the present invention can be employed in imaging systems using a variety of imaging modalities, such as radiography and magnetic resonance. The markers and phantoms also have particular utility within MRI-Linac systems, which combine both magnetic resonance imaging and radiotherapy within the same system.

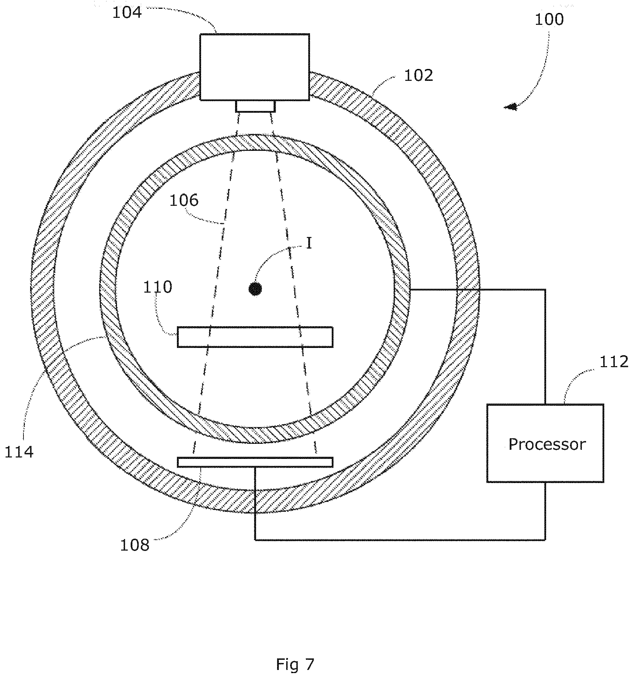

Such a system 100 is shown schematically in Figure 7.

The system comprises a gantry 102, which is able to rotate about an axis I. A radiation head 104 is mounted to the gantry, and adapted to generate a beam of radiation 106 directed substantially inwards towards the rotation axis I. A source of radiation (such as a linear accelerator, or a radioisotope) may be provided to generate the radiation which emanates from the head 104. In order to have a therapeutic effect, the energy of the radiation beam will typically be in the order of megaelectronvolts. A patient support 110 is provided on or near the rotation axis I, on which a patient or an object to be imaged can be placed.

The shape and direction of the beam 106 can he adapted by the use of collimators such as a multi-leaf collimator (not illustrated), to conform to a particular desired profile.

For example, the shape of the beam may be adapted to conform to the profile of a target structure within a patient on the patient support 110. As the gantry rotates, the radiation beam 106 is directed towards the target structure from multiple directions and dose builds up in the target structure while being generally reduced in the surrounding areas.

A radiation detector 108 is mounted on the gantry 102 at a position substantially opposite the radiation head 104, such that the radiation beam 106 impacts the radiation detector 108 after passing through an object to be imaged on the patient support 110.

Such detectors are often called portal imagers as they generate a projection image of the object along the axis of the radiation beam 106. The detector 108 is coupled to processing circuitry 112 which uses the detection data to produce an image. Thus, although the radiation system is primarily used for therapeutic purposes and generates radiation at an energy suitable for therapy, the radiation can nonetheless be used to generate images (albeit at lower contrast than the less energetic radiation conventionally used for imaging).

The system 100 further comprises a magnetic resonance imaging apparatus. Those skilled in the art will appreciate that such an apparatus comprises a large number of components, including various magnetic coils for generating specific magnetic field strengths at specific locations and an RF system for transmitting and receiving electromagnetic waves. Only the magnetic coils 114 are illustrated here for clarity.

The magnetic coils 114 of this example are positioned with their longitudinal axes aligned with the rotational axis I of the gantry 102 (other geometries are possible). In one embodiment, the coils 114 are displaced from each other along the direction of the axis I such that a gap is created. The radiation beam 106 can he directed through this gap such that the magnetic coils 114 do not interfere with the radiation. The coils 114 are coupled to the processing circuitry 112 such that an image can be produced of an object on the patient support 110.

An object which is placed on the patient support 110 can therefore be imaged by the MRI system and treated by the radiotherapy system while in the same position.

Further, the radiotherapy system can be used to generate a portal image of the object using the radiation detector 108. The markers 20, 30 and phantoms 50, 60 described above can be used to calibrate the system 100 such that imaging data collected by the MRI system can be used to inform and control the radiotherapy system.

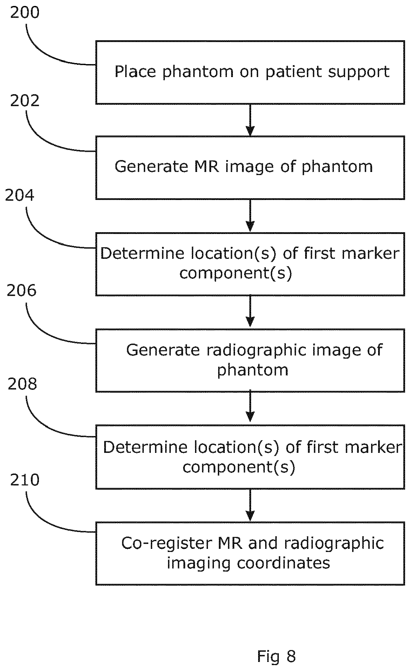

Figure 8 is a flowchart describing a method of calibrating the system 100 described above.

In step 200, a phantom according to embodiments of the invention is placed on the patient support 110, in the path of the radiation beam 106 and at a position suitable for imaging by the MRI system. The phantom may be a single marker 20, 30 as shown in Figures 3, 4 or 5, or a plurality of such markers as shown in Figures 6a and 6b.

In step 202, the MRI system is used to generate an image of the phantom, and particularly used to generate an image showing the locations of the one or more second marker components. In this way, the first marker component can also be imaged because of the common boundary between the two component types.

In step 204, the locations of the one or more first marker components are determined from the MRI image.

In step 206, a radiographic image is generated of the phantom using the radiation beam 106 and the radiation detector 108 while in the same position. In this image, the one or more first marker components show more clearly due to their higher material density.

In step 208, the locations of the one or more first marker components are determined from the radiographic image.

In step 210, the knowledge of the first marker component locations in both images allows the coordinate systems of the MR imaging modality to be co-registered with the coordinate system of the radiographic modality. In particular, measurements using the MRI system can be used to instruct the radiotherapeutic system. For example, the positions of the collimating elements may be adapted on the basis of MR imaging data in order to track a moving target.

Embodiments of the present invention therefore provide markers, phantoms, and associated methods of calibration which are suitable for use in a wide variety of medical imaging systems.

It will of course be understood that many variations may be made to the above-described embodiment without departing from the scope of the present invention.

**Claims**

1. A marker for use in calibration of a radiotherapeutic apparatus, comprising:

   a first component having a first hydrogen proton density and a first mass density, and being solid;
   a second component having a second hydrogen proton density and a second mass density, and being liquid,
   wherein the first component is substantially completely surrounded by the second component within a void of the second component to form an interface between the first and second components;
   the marker further comprising a housing enclosing the first and second components, and defining a region into which the second component can extend at a location which is displaced vertically from the interface between the first and second components, thereby to allow trapped air to be collected.

2. A marker according to claim 1 in which the housing further comprises a restraint for restraining the first component in a pre-determined position within the housing.

3. A marker according to claim 2 in which the restraint and the region form discrete portions of the housing.

4. A marker according to any one of the preceding claims in which the housing comprises a tapered portion thereby to define the region.

5. A marker according to any one of the preceding claims in which the restraint is formed by a shape of the housing.

6. A marker according to claim 5 in which the shape is a tapered shape.

7. A marker according to claim 6 as dependent on claim 4 wherein the tapered shape is that of the tapered portion.

8. A marker according to any one of claims 1 to 4 in which the restraint comprises a portion which extends into the marker.

9. A marker according to any one of the preceding claims in which the restraint comprises a region of the marker which is narrower than the housing.

10. A marker according to any one of the preceding claims in which the restraint comprises one or more fingers or tongues extending from the housing into the marker, thereby to allow fluid flow around it or them while inhibiting movement of the first component.

11. A marker according to any one of the preceding claims in which the first component is spherical.

10

14      12

16

Fig 1 - PRIOR ART

10

Fig 2a

10

Fig 2b

Fig 3

Fig 4

Fig 5

50    52

54

54

Fig 6a

60    62

64    Fig 6b

Fig 7

200

Place phantom on patient support

202

Generate MR image of phantom

204

Determine location(s) of first marker component(s)

206

Generate radiographic image of phantom

208

Determine location(s) of first marker component(s)

210

Co-register MR and radiographic imaging coordinates

Fig 8

Europäisches
Patentamt

European
Patent Office

Office européen
des brevets

# EUROPEAN SEARCH REPORT

Application Number

EP 19 16 4169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAMES O'CALLAGHAN ET AL: "Is Your System Calibrated? MRI Gradient System Calibration for Pre-Clinical, High-Resolution Imaging", PLOS ONE, vol. 9, no. 5, 7 May 2014 (2014-05-07), page e96568, XP055305468, DOI: 10.1371/journal.pone.0096568 | 1-10 | INV. G01R33/58 G01R33/48 |
| Y | * Chapters "Gradient Calibration Protocol Description" and "Methods: 3D Grid Phantom"; page 2; figures 2,3 * | 11 | |
| X | US 2015/323639 A1 (BOSS MICHAEL A [US]) 12 November 2015 (2015-11-12) * paragraphs [0062] - [0072]; figures 1-14 * | 1-10 | |
| Y | GB 2 512 416 A (ELEKTA AB [SE]) 1 October 2014 (2014-10-01) * abstract; claims 1-11; figures 1,3,7 * | 1-11 | |
| Y | DE 10 2005 045679 B3 (BRUKER BIOSPIN MRI GMBH [DE]) 1 March 2007 (2007-03-01) * paragraphs [0007] - [0028], [0042] - [0046]; figures 1,2b, 3, 4a-4c * | 1-3,5, 8-10 | TECHNICAL FIELDS SEARCHED (IPC) G01R A61B |
| A | | 4,6,7,11 | |
| Y | JP S56 176708 U (HITACHI MEDICAL CORPORATION) 26 December 1981 (1981-12-26) * figures 1,3,4 * | 1-11 | |

The present search report has been drawn up for all claims

| Place of search | Date of completion of the search | Examiner |
|---|---|---|
| Munich | 19 August 2019 | Faber-Jurk, Sonja |

EPO FORM 1503 03.82 (P04C01)

**ANNEX TO THE EUROPEAN SEARCH REPORT
ON EUROPEAN PATENT APPLICATION NO.**

EP 19 16 4169

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2019

| Patent document cited in search report | | Publication date | Patent family member(s) | | Publication date |
|---|---|---|---|---|---|
| US 2015323639 | A1 | 12-11-2015 | NONE | | |
| GB 2512416 | A | 01-10-2014 | CN | 105658168 A | 08-06-2016 |
| | | | EP | 2996614 A1 | 23-03-2016 |
| | | | GB | 2512384 A | 01-10-2014 |
| | | | GB | 2512416 A | 01-10-2014 |
| | | | US | 2016038116 A1 | 11-02-2016 |
| | | | WO | 2014154861 A1 | 02-10-2014 |
| DE 102005045679 B3 | | 01-03-2007 | DE | 102005045679 B3 | 01-03-2007 |
| | | | GB | 2433785 A | 04-07-2007 |
| | | | US | 2007069725 A1 | 29-03-2007 |
| JP S56176708 | U | 26-12-1981 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2512416 A